# EUROPEAN PATENT SPECIFICATION

(11) **EP 1 637 182 B1**
(45) Date of publication and mention of the grant of the patent: **16.02.2011**
(21) Application number: 03782845.6
(22) Date of filing: 19.12.2003
(51) Int. Cl.: A61N 5/067, A61N 5/06

(54) **PHOTODYNAMIC THERAPY APPARATUS**
PHOTODYNAMISCHES THERAPIEGERÄT
APPAREIL DE TRAITEMENT PHOTODYNAMIQUE

(30) Priority: 20.06.2003 JP 2003176687
(43) Date of publication of application: 22.03.2006
(73) Proprietor: Keio University, Tokyo 108-8345 (JP); Terumo Kabushiki Kaisha, Tokyo, 1510072 (JP)
(72) Inventor: ARAI, Tsunenori, Keio University, Yokohama-shi, Kanagawa 223-0061 (JP); OHMORI, Sayaka, Keio University, Yokohama-shi, Kanagawa 223-0061 (JP); YANAGIHARA, Takeshi, Keio University, Yokohama-shi, Kanagawa 223-006 (JP)
(74) Representative: Casalonga, Axel
(86) International application number: PCT/JP2003/016344
(87) International publication number: WO 2004/112902

(56) References cited:
- EP-A1- 1 101 450
- WO-A-02/36200
- WO-A-91/18646
- WO-A-93/21842
- WO-A-99/63900
- WO-A1-00/59505
- WO-A1-01/15694
- WO-A1-84/00101
- WO-A1-95/05214
- WO-A1-97/02862
- WO-A1-98/18399

## Description

### Technical field

The present invention relates to photodynamic therapy equipment . More particularly, the present invention pertains the photodynamic therapy (superficial lesion preserving therapy) equipment, which can damage the deep-lying lesioned part with preserving the normal superficial region, without damaging, covered on the lesioned part in the treatment of the deep-lying lesioned part in the body .

### Background art

Photochemical therapy (PDT; also designates as photodynamic therapy) is considering the possibilities of applying to various treatments in addition to the endoscopic phototherapy of early carcinoma. PDT is a therapeutic method, in which the photosensitive substance (photo-sensitizer) such as certain types of porphyrin derivatives is administered by a method of intravenous injection, subjected to adsorb and accumulate selectively in lesioned tissues such as cancer tissues to be treated, and then the light such as laser beam is irradiated to damage the lesioned tissues.

PDT is utilized the property accumulating the photo-sensitizer selectively to the lesioned part and the property sensitized by the light. A mechanism of action, in which the photo- sensitizer incorporated into the lesioned part is excited by the light irradiation to generate the active singlet oxygen as a result of transferring the energy of the sensitizer to the oxygen in the lesioned part, and the active oxygen necrotizes cells in the lesioned part, is proposed.

Various types of porphyrin used in the photodynamic therapy have been reported (JP,09-124652,A(1997); WO 98/14453; JP, 04-330013,A(1992); and JP, 2961074,B) . Various cancers (JP,07-53733,B(1995); JP,09-124652,A(1997)), autoimmune diseases (WO 99/07364; WO 98/19677; WO 98/14453) and arteriosclerosis(JP,3154742,B;WO00/59505)are reported as diseases for applying photodynamic therapy.

In the photodynamic therapy, the porphyrin derivative developed in the initial stage exhibited the short absorption wavelength as 600 nm. Consequently, there was a problem that even if the laser of this wavelength range was irradiated, the beam could only arriving at the depth to several millimeters from the surface, and the deep-lying lesioned part could not be treated. Actually, since the laser practically used in PDT is the laser with short wavelength and low power for not damaging the normal tissue, the penetration depth is only several millimeters and is used targeting for treatment of superficial early stage cancer.

There was further problem that drugs developed in the early stage exhibited no good excretion from the body and photosensitivity complications (sunburn symptom).

Second generation drug having absorption wavelength toward longer wavelength with superior excretory property was developed (JP,05-97857,A(1993); JP,06-80671,A(1994)) for reducing the problem on "photosensitivity disease" and providing possibility of treating deep-lying lesioned part. However, although such the second generation drug for PDT was developed, the technologies for controlling parameters for laser irradiation have not established and matters in question such as optimum intensity of laser irradiation and irradiation time have not been elucidated.

As described above, since the second generation PDT drug is expected to apply for the deep-lying lesioned part, PDT combined with the second generation drug, which has possibility for treating from the superficial to the deep-lying lesion, and the long wavelength laser may be effective for treatment of cancer lesion invaded deeply due to progressing superficial cancer. In case of the deep-lying lesioned part with normal surface region, which is not applied only to cancer, damage of the healthy surface region might be expected.

For that reason, in case of normal surface region with deep-lying lesioned part, highly invasive treatment with irradiating the laser beam directly to the lesioned part by inserting the laser irradiation part into the tissue was only studied, and a development of PDT with low invasive method for damaging the deep-lying lesion has been thought to be difficult.

WO 93/21842A describes a photodynamic therapy system according to the preamble of claim 1.

The present invention is intended to provide the photodynamic therapy (superficial part preserving therapy) equipment, which is able to control the treatment depth from the superficial part to the deep-lying part of the lesion by changing the laser irradiation condition and is able to damage the deep-lying lesioned part with preserving the normal superficial region, without damaging, covered on the lesioned part .

### Disclosure of the invention

The inventors of the present invention have extensively studied PDT drugs and the irradiation condition of the pulse laser such as a type of the laser medium, the peak intensity of the laser and repetition frequency. As a result, we have found that although therapeutic efficiency of PDT (damaged rate of the lesioned tissues) was increased as the peak intensity was increased when the peak intensity of the irradiating beam was increased from the low intensity to the high intensity, the therapeutic efficiency was inversely reduced when the peak intensity became higher.

Namely, we have found that when the laser irradiation peak intensity was plotted on the horizontal axis and the therapeutic efficiency of PDT was plotted on the vertical axis, the efficiency of PDT was increased to the upward direction depending on increasing the peak intensity and the efficiency of PDT was decreased to the downward direction depending on increasing the peak intensity after attaining to the highest efficiency in certain depth in the graph indicating the efficiency of PDT. This phenomenon means that the PDT therapy is valid when the peak intensity of the irradiating laser beam falls within the certain range, and the therapeutic effectiveness is reduced when the peak intensity becomes larger or smaller than that.

We have further studied the irradiation condition of laser beam and the therapeutic efficiency of PDT based on such novel knowledge. As a result, we have found that when the laser beam of the high peak intensity likely reaching to the deep-lying part of the body was irradiated to the lesioned part of the living body, although the peak intensity was high at the time point of entering laser beam into the body, the peak intensity of the laser beam was gradually dropped due to absorption of the laser energy by the accumulated PDT drug in the lesioned part and hemoglobin in the body, and the peak intensity was dropped down when the irradiated laser beam was arrived at the deeper position.

Further we have found that the peak intensity of the laser beam could be maintained within the range of the high therapeutic efficiency of PDT in the certain range of the depth and the PDT therapy could be achieved in that depth. Contrary to these findings, it is suggested that when the PDT therapy is intended to perform effectively at the fixed depth, an effect of PDT is made to increase upward by adjusting the peak intensity of the irradiating laser beam when the laser beam is reached to that depth.

Further, we have studied the principle of PDT therapy, in which the energy of the PDT drug excited by the laser irradiation was transferred to oxygen atoms in the tissues to generate active oxygen causing to damage surrounding cells, and the PDT therapy, in which the irradiation depth of laser beam could be controlled.

As a result, we have found that since a fixed amount of time was required until oxygen atoms, which had been consumed locally at the position of the area irradiated by the laser irradiation, were diffused in and supplied from the surrounding tissues, an irradiation timing of the laser beam should be adjusted to the oxygen supply. When the repetition frequency of the irradiated laser beam was altered in order to change the irradiation timing of the laser beam, it was found that the therapeutic efficiency depended upon not only the peak intensity of the laser but also the repetition frequency of the laser.

Further, we have examined that the deep-lying lesion alone might be able to damage without damaging the superficial normal region by controlling the therapeutic depth, for example, in case of the disease, in which the lesioned part was covered with the normal region as like the lesion of arterial sclerosis, and found that the deep-lying lesion alone could be damaged without damaging the superficial normal region by performing irradiation of the pulse laser of the high peak intensity.

We have further examined and completed the appropriate controlling system for laser irradiation and the catheter equipment which could be applied to PDT and treated the lesioned part locally.

Aspects of the present invention are defined in the appended claims

### Brief explanation of drawing

Fig. 1 is a graph showing conceptually a relationship between the peak intensity of pulsed light and PDT efficiency.
Fig. 2 is a graph showing conceptually a relationship between decrease of the peak intensity of light in case of irradiating the light to tissues and a region of depth with favorable efficiency of PDT.
Fig. 3 is a graph showing conceptually a relationship between rate of cell death and depth.
Fig. 4 is a diagram showing schematic construction of equipment for treating arteriosclerosis of the present invention.
Fig. 5 is a schematic diagram showing photodynamic therapy equipment equipped with balloon on the tip of inserted part to the body.
Fig. 6 is a flow chart showing flow in use of equipment of the present invention.
Fig. 7 is a drawing showing peak intensity and repetition frequency dependency of PDT effect.
Fig. 8 is a drawing showing relationship between drug breaching and peak intensity.
Fig. 9 is a drawing showing change of rate of cell death when irradiation energy density is changed.
Fig. 10 is a drawing showing measurement result of rate of cell death to depth in each pulse energy density when pulse number is maintained constant.
Fig. 11 is a drawing showing measurement result of rate of cell death to depth in each pulse energy density when amount of total irradiating energy is maintained constant.
Fig. 12 is a drawing showing measurement result of rate of cell death to depth when pulse energy density is changed during irradiation.

### Best mode for carrying out the invention

The present invention will be explained hereinbelow in detail.

The present invention provides laser irradiation equipment used for PDT and equipment which is able to control therapeutic depth, i.e. depth of damaged region, by changing the laser irradiation condition.

Further, the present invention provides equipment, which makes possible to treat lesioned part by damaging only the deep-lying lesioned part (superficial lesion preserving therapy) with preserving the superficial normal region without damaging, when the lesioned part exists in the deep region of the tissue or the lesioned part is covered with normal membrane.

PDT (photodynamic therapy) herein means the therapeutic method for damaging and disrupting selectively the lesioned tissues by accumulating the photosensitive substance (PDT drug) having affinity to the specific lesioned part to the lesioned part specifically, and irradiating the light having specific wavelength.

The lesioned part targeted for treatment by the laser irradiation equipment of the present invention is the lesioned part of the disease accompanied by abnormal cell growth in the tissue or atherogenesis, and the lesioned part, for which progress of the disease is terminated to perform treatment or to prevent expansion by damaging the tissue lesion. Examples of disease having such the lesioned part are cancer, sarcoma, benign tumor and arteriosclerosis accompanied by atheroma.

Position of generation of diseases is not limited, and progressive level is also not limited. For example, in case of cancer, cancers from superficial early cancer to invasive advanced cancer are included. Among them, the disease infiltrating the lesion to deep tissues, and the disease, in which the lesion is covered by the normal tissues, is more suitable for the present invention.

Normal tissues covering the lesioned part is not limited to the same tissue of the lesion, and when the light is intended to irradiate to the lesioned part by using the equipment of the present invention, any cases, wherein the lesioned part and the region to be irradiated with light are coexisted, can be included.

Examples of such disease are intraepithelial cancer having normal epithelial surface, interstitial (interstitial cell: cells constituting the supporting tissue) cellular sarcoma existing within the tissue and sarcoma covered with the epithelial cells, disease having normal part (urethral wall) between the lesioned part (prostate) and the light irradiating part, when the light irradiation part is inserted into the urinary tract in case of prostate cancer and prostate hyperplasia, and disease having normal part (capsule) which covers the lesioned part (atheroma) between the lesioned part (atheroma) and the light irradiating part, when the light irradiation part is inserted into the artery in case of atherosclerosis.

An embodiment of the equipment of the present invention is catheter-like equipment with light irradiating part. Equipment of such embodiment can be used by inserting into the tube of tubular tissue such as urinary tract and blood vessel, and is used preferably for treatment of prostate cancer, prostate hyperplasia, arteriosclerosis, bladder cancer, esophageal cancer, rectal cancer, larger intestinal cancer, cervix carcinoma, cancer of uterine body, carcinoma of the biliary tract and pancreas cancer.

Depth of the superficial part and the deep part of the present invention is not limited, and the superficial part means the depth of about 0.05 mm to 10 mm, 0.05 mm to 7 mm, 0.05 mm to 5 mm, 0.05 mm to 3 mm, or 0.05 mm to 1 mm from the light irradiated surface, and the deep part means the deeper part that the above.

As like the case of the urethral wall and the prostate, even in case of different tissues in the lesioned part, which is intended to be treated by damaging tissues, and the normal part, which is intended to be preserved without damaging, the normal part existing between the lesioned part and the light irradiation means is called as the superficial part, and the thickness thereof is about 0.05 mm to 10 mm, 0.05 mm to 7 mm, 0.05 mm to 5 mm, 0.05 mm to 3 mm, or 0.05 mm to 1 mm. For example, the thickness of the urethral wall, which is to be preserved, for treating prostate cancer and prostate hyperplasia, is about 0.5 mm to 2 mm, and in case of treating atherosclerosis, the thickness of the membrane to be preserved, which covers atheroma to be disrupted, is about 0.05 mm to 0.2 mm.

In PDT, the photosensitive substance, which can be accumulated in the lesioned part, should be administered. The PDT drug combined with the equipment of the present invention is not limited to the specific compound and known PDT drug can be used in combination with the light irradiating the absorption wavelength of the compound. PDT drug and type of the light (species of light source, wavelength of light, etc. ) may be selected depending upon the depth of the lesioned part.

PDT drug used in the PDT in practice at present is porfimer sodium (PHE) with absorption wavelength 630 nm, which is used in combination with 630 nm excimer laser. However, since the invasion depth of the light irradiated by the excimer laser is about 2 to 3 mm, it is limited for treatment of superficial cancer.

Since the equipment of the present invention can be used for treatment of the lesioned part in the deep part, which is impossible by using the present PDT, the laser light with long wavelength for larger invasion depth can be used. Consequently, not only PDT drug having absorption wavelength at about 630 nm but also the drug having long wavelength range can be used. Among them, second generation drug having absorption wavelength at 650 nm to 800 nm is preferably used. In addition, the second generation drug has good excretory nature from the body and the drug is further recommended in this point.

Examples of the second generation drug are: coumalin based drug, ATX-S10 (670 nm) (Iminochlorin aspartic acid derivative, Toyo Hakka Kogyou K.K., transferred the right to Hikari Chemicals Labs. in 2000, JP, 06-80671,A (1994)); NPe6 (664 nm) (mono-L-aspartyl chlorine e6, JP,2961074,B); mTHPC (652 nm) ; SnET2 (660 nm) (tin etiopurpurin, Miravant Medical Technologies); AlPcS (675 nm) (chloro aluminium sulphonated phthalocyanine); BPD-MA (690 nm) (benzoporphyrin derivative monoacid ring A, QLT Inc.); and Lu-tex (732 nm) (Lutetium Texaphyrin) (traditional term, absorption peak wavelength, general name, place to obtain, and reference, in this order) . These drugs can be used in combination. Not only the light wavelength and repetition wavelength but also irradiation light intensity can be controlled by accumulating multiple drugs having different absorption wavelength, and broader range of lesioned part from the superficial part to the deep part can be treated.

Administration of the drug can be performed by dissolving the drug in proper buffer solution such as phosphate buffer saline, and if necessary, pharmaceutically acceptable additives are added. Examples of additives are solubilizing agent, pH adjuster such as acid and alkali base, tonicity agent such as ascorbic acid, diluting agent such as glucose, and isotonic saline such as sodium chloride.

Route of administration is not especially limited. It can be administered by intravenous injection, intramuscular injection, subcutaneous injection, and oral administration. For reducing photosensitivity disease, it may be administered directly to the lesioned part. For example, in case of disease of arteriosclerosis and prostate hyperplasia to be treated, drug can be administered locally by using the drug delivery catheter, which is equipped with the vascular catheter and the urethral catheter, drug administration means such as needle and drug injection device. In the conventional PDT, PDT drug is mainly administered intravenously, and in that case, in order to accumulate PDT drug mainly in the lesioned part and to emphasize the contrast of the accumulated PDT drug between the normal region and the lesioned part (generally, about six-fold PDT drug per unit volume is accumulated in the lesion as compared with the normal region) , 48 to 72 hours are required for the light irradiation after intravenous administration, and patient had to become strained largely.

This is due to relying upon only the accumulation of the drug for selectivity of the range of PDT therapy in the conventional method, and to removal of PDT from the normal region for avoiding damage of the normal region. Especially, when the lesioned part is covered by the normal region, PDT drug should be removed completely from the normal region for avoiding damage of the normal region, but this is practically impossible, and in the conventional method, treatment, in which only the deep lesion is damaged and the superficial part is preserved, is impossible.

In the PDT equipment, which can control the therapeutic depth, of the present invention, since the range of PDT mainly depends on the condition of laser irradiation, PDT drug should not always to be removed from the normal region and efficient treatment can be expected. Consequently, after administration of PDT drug, completion of the contrast image observed by the accumulation of PDT drug in the normal region and the lesioned part should not be waited, and the light irradiation can be initiated immediately after administration of PDT drug or within short period of time.

Amount of administration of PDT drug is not limited, and in case of systemic intravenous administration, etc., it is 0.01 to 100 mg/kg body weight, preferably 1 to 5 mg/kg body weight. In case of local administration, for example, a drug formulated in several µg/ml to several mg/ml, a preparation, several µl to several ml, may be directly administered by infusion into the lesioned part. As explained later, since the degree of accumulation of the drug in the lesion can be monitored by the equipment of the present invention, the drug can be administered additionally according to the observation in monitoring.

Types of light species irradiated for treatment in the equipment of the present invention are not limited, continuous or pulsating laser beam or the light generated by the optical parametric oscillator (OPO) with variable wavelength is preferable.

The wavelength irradiated is 600 nm to 800 nm, and the light beam with the wavelength near to the absorption wavelength of PDT drug in use can be applied. Especially, the wavelength of the light generated from OPO can be freely varied, and it can be treated the lesion broadly from the superficial part to the deep part by changing the wavelength and peak intensity of the irradiating light.

Examples of laser beam preferably used are semiconductor laser beam, dye laser radiation and second harmonic waves of variable wavelength near-infrared laser beam. The light can be the pulsed light such as pulsating laser beam or continuous light beam such as continuous laser beam. The pulsed light means pulse width below 1 ms. The continuous light can be irradiated as pulsating chopped light intermittently by using light chopper. When continuous light such as continuous laser beam is irradiated, if the peak intensity exceeds above the constant value, the irradiating part is deformed by heating, as a result it is not suitable for the superficial lesion preserving therapy by performing the high peak intensity irradiation. Consequently, when the superficial lesion preserving therapy is conducted, pulsed light is preferably used.

When the deep-lying lesion alone is treated with preserving the superficial part, the pulsed light with high peak intensity is irradiated. The superficial normal region can be preserved without damage by irradiating the high peak intensity pulsed light. In case that the high peak intensity pulsed light is irradiated into the living body tissues, even if PDT drug is accumulated, the deep-lying lesion can be damaged without damaging the superficial tissues.

When the low peak intensity light is irradiated to the tissue, in which PDT drug is accumulated, the superficial part of the tissue is damaged. When the high peak intensity light is irradiated, the light further proceeds to the deep part, the more light energy is absorbed and scattered by the accumulated PDT drug in the tissue and hemoglobin in the tissue, and the peak intensity of the pulsed light is attenuated, then when it reaches to the certain constant depth, the PDT effect is increased to damage the tissue in that depth.

Namely, although the superficial part is not affected damage by irradiation of the high peak intensity pulsed light, the deep part alone is damaged. In case that the lesioned part is broadly distributed from the superficial part to the deep part, the condition of irradiating light may be altered. Namely, the continuous light or pulsed light with the low peak intensity is irradiated for treatment of the superficial lesionedpart, and the pulsed light with the high peak intensity is irradiated for treatment of the deep-lying lesion.

The condition of light irradiation may preferably be determined depending on the size of the lesioned part, type of light used and PDT drugs. Relationship between the peak intensity of light and the therapeutic depth can be estimated easily by using the model mimicked to the tissue (e.g. the model prepared by using animal tissues).

A unit of the peak intensity of irradiating light is W/cm². Further, in case of performing PDT by irradiating light, the pulse energy density (irradiation dose, J/cm²) determines the feasibility of PDT, and the peak intensity or the pulse energy density can be determined properly according to the condition of the lesioned part. The pulse energy density can be obtained by multiplying the peak intensity of light with the pulse width, i.e. pulse energy density = peak intensity × pulse width.

In the peak intensity of irradiating light, range of the high peak intensity and that of the low peak intensity are not limited, and are determined properly depending on the type of light, the depth of lesion to be treated, etc.

Even in case of irradiation with setting the light irradiation part close to the lesioned part by using equipment with the catheter as described later, and irradiation from outside of the body, ranges of the high peak intensity and the low peak intensity are different. For example, when the irradiation is performed to the lesioned part, where PDT drug is accumulated from the superficial part to the deep part, the light with peak intensity which can damage the superficial part such as 0.05 mm to 10 mm, 0.05 mm to 7 mm, 0.05 mm to 5 mm, 0.05 mm to 3 mm, or 0.05 mm to 1 mm from the surface, is designated as the light of low peak intensity, and the light with peak intensity which can damage deeper area than the above is designated as the light of high peak intensity.

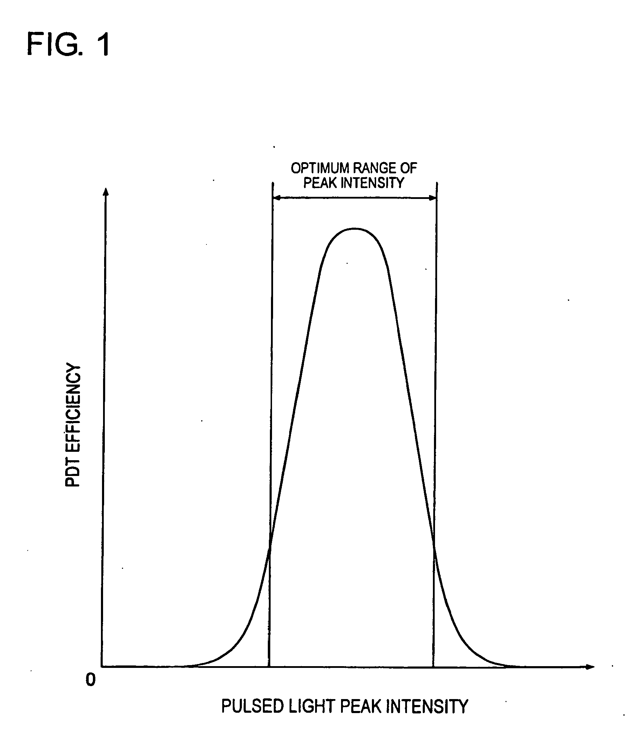

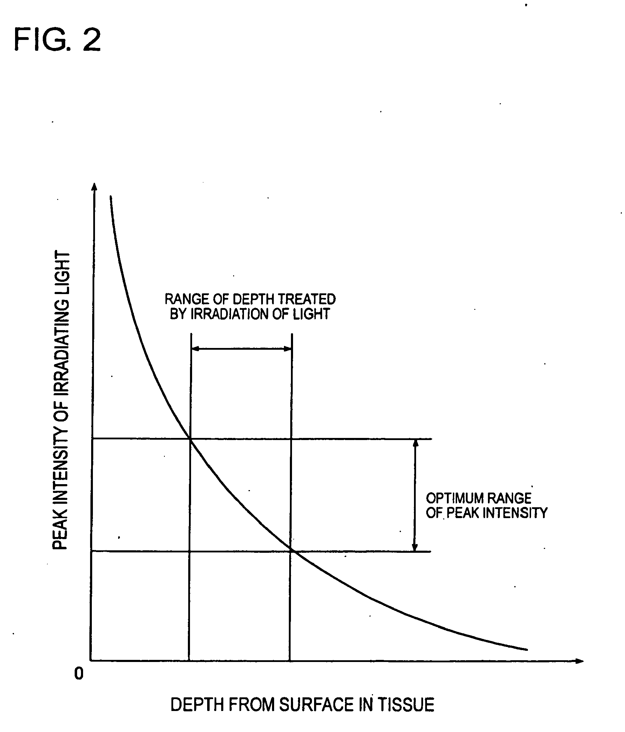

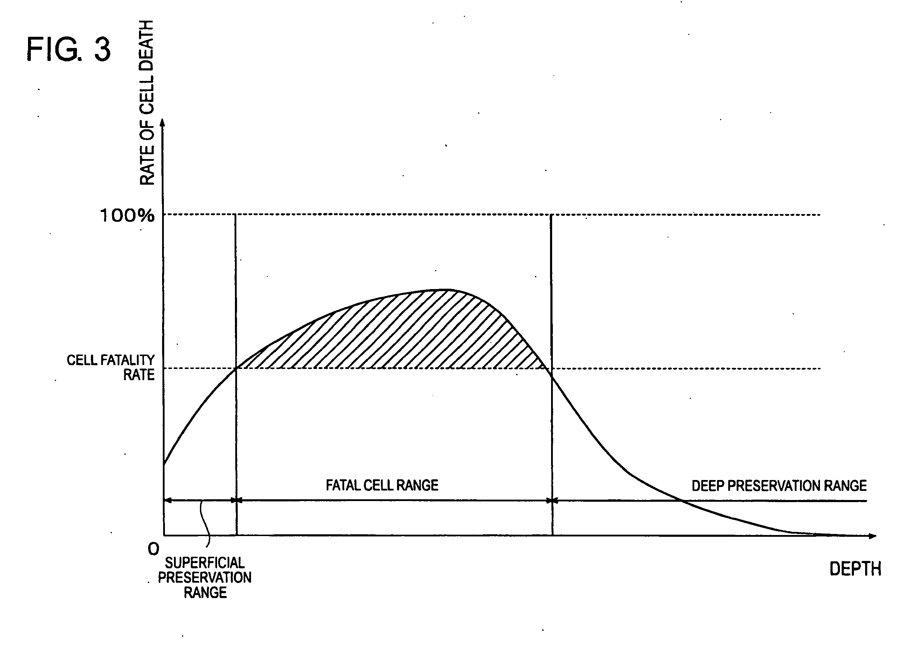

Fig. 1 is a drawing showing conceptually a relationship between the peak intensity of light and PDT efficiency. Fig. 2 is a drawing showing conceptually a relationship between decrease of the peak intensity of light in case of irradiating the light to tissues and a region of depth with favorable efficiency of PDT. Fig. 3 is a drawing showing conceptually a relationship between rate of cell death and depth.

Referring to the conceptual drawing, the range of the peak intensity of the light of the low peak intensity for treating the superficial part and the light of the high peak intensity for treating the deep part in the tissue intended to be treated can be determined. The peak intensity of the irradiating light can be mentioned as the range of 100 mW/cm² to 5 MW/cm². Total energy density which can be exemplified is 20 to 500 J/cm².

Example of the peak intensity of the light with high peak intensity is ranging from 10 kW/cm² or more to below the threshold value in which generating plasma on the surface of the body by the pulse irradiation. The peak intensity of the light with high peak intensity is ranging from 100 kW/cm² to 10 MW/cm². More preferably, the peak intensity of the light with high peak intensity is ranging from 200 kW/cm² to 5 MW/cm².

As shown in Fig. 1, in case that the peak intensity of the pulsed light is within the range of the optimum peak intensity, the photosensitive substance is activated and efficiency of the photodynamic therapy is high. In case that the peak intensity of the pulsed light is higher than the range of the optimum peak intensity, or lower than the range of the optimum peak intensity, the therapeutic efficiency is low and the photosensitive substance is not activated. The photodynamic therapy equipment and method of the present invention utilizes such property.

As shown in Fig. 2, the peak intensity of the pulsed light is attenuated during the way of passing through the body. Consequently, in the superficial part, which was just irradiated to the body, even if the peak intensity is higher peak intensity than the range of the optimum peak intensity, it attenuates gradually to the range of the optimum peak intensity, further to attenuate to lower peak intensity than the range of the optimum peak intensity. By utilizing this, when the light arrives at the predetermined depth of the body, the peak intensity of the initially irradiating light can be adjusted to the peak intensity within the range of the optimum peak intensity.

In the body, to which the light with the peak intensity within the range of the optimum peak intensity hereinabove described is irradiated, a rate of cell death becomes above the cell fatality rate as a result of activation of the photosensitive substance. The rate of cell death means the rate of cells damaged by an action of activation of the photosensitive substance. Further, the cell fatality rate means a criterion for the rate of cell death, in which function of the organ becomes unrecoverable by an action of the photosensitive substance. The cell fatality rate is different depending on type of organs.

As described above, since the light proceeds into the body with attenuating, the rate of cell death is changed depending on the depth. Mode of this change is shown as in Fig. 3.

As shown in Fig. 3, in the part, where the light with the peak intensity within the range of the optimum peak intensity, a range of the cell fatality is constituted, and in the anteroposterior regions thereof, the superficial preserved region and the deep preserved region are formed.

The range of cell fatality is the range where the rate of cell death exceeds the cell fatality rate. The superficial preserved region is the range, in which the photosensitive substance is not activated and the rate of cell death is below the cell fatality rate, since it is shallower than the range of cell fatality and the peak intensity of passing light is higher than the range of the optimum peak intensity. The deep preserved region is the range, in which the photosensitive substance is not activated and the rate of cell death is below the cell fatality rate, since it is deeper than the range of cell fatality and the peak intensity of passing light is lower than the range of the optimum peak intensity.

In the photodynamic therapy, the lesion is treated by matching the range of the lesion and the range of cell fatality. The healthy region shallower than the lesioned part is preserved as the superficial preservation range and the healthy region deeper that the lesioned part is preserved as the deep preservation range.

When the pulsed light is used as the light for irradiating the body, the repetition frequency of the irradiating pulsed light should be adjusted for increasing the PDT efficiency. This is due to as follows. The energy of PDT drug excited by the irradiation of light is transferred to the surrounding oxygen atoms and the oxygen is converted to active oxygen and exert its action to the cells, and as a result, the oxygen concentration of the light irradiated area is temporarily lowered, then the subsequent irradiation of pulsed light has to be waited until the oxygen is supplied from the surrounding tissues by diffusion.

Namely, if the repetitive frequency is too high, oxygen supply becomes too late and therapeutic efficiency of PDT is decreased, and if the repetitive frequency is too low, the irradiation time of the light becomes too long and the PDT is impossible to perform. Consequently, the repetition frequency, by which preferable therapeutic efficiency can be obtained, has defined range.

The repetition frequency can preferably be changed depending upon the oxygen concentration in the area to be treated and accumulated amount of PDT drug, and the proper repetition frequency can be defined by using the model mimicking the tissues as described hereinabove. Range of the repetition frequency is not limited and is for example 1 Hz to 1 kHz.

As described above, since the repetition frequency affects to PDT efficiency, the PDT efficiency can be improved by maintaining the repetition frequency within the constant range.

Also, as explained hereinbelow, since the equipment of the present invention is possible to monitor amount of PDT drug and oxygen concentration of the area to be treated, the peak intensity and the repetition frequency of the irradiating light can preferably be adjusted depending on the amount of PTD drug and/or oxygen concentration.

The PDT drug excited by irradiation of light is decomposed by an action of active oxygen (bleaching). Consequently, in case that treating the lesion reaching to the deep part and the lesion, in which the drug is evenly accumulated, when the light with the low peak intensity is irradiated for the first time, PDT drug in the superficial part is successively decomposed with exhibiting the therapeutic effect. Subsequently, when the light of the high peak intensity is irradiated, PDT drug in the superficial part has already been decomposed to loose activity, and the irradiated light is reached to the deep part without being absorbed by the PDT drug in the superficial part and the treatment of the deep lesion can be achieved efficiently.

In order to treat from the superficial part to the deep part, treatment is performed by irradiating the light with low peak intensity at the beginning, subsequently the peak intensity of the light is preferably increased gradually. In this case, for example, the peak intensity may be changed stepwisely depending on the progress of the irradiation time or the peak intensity may be changed continuously.

### (Equipment of the present invention)

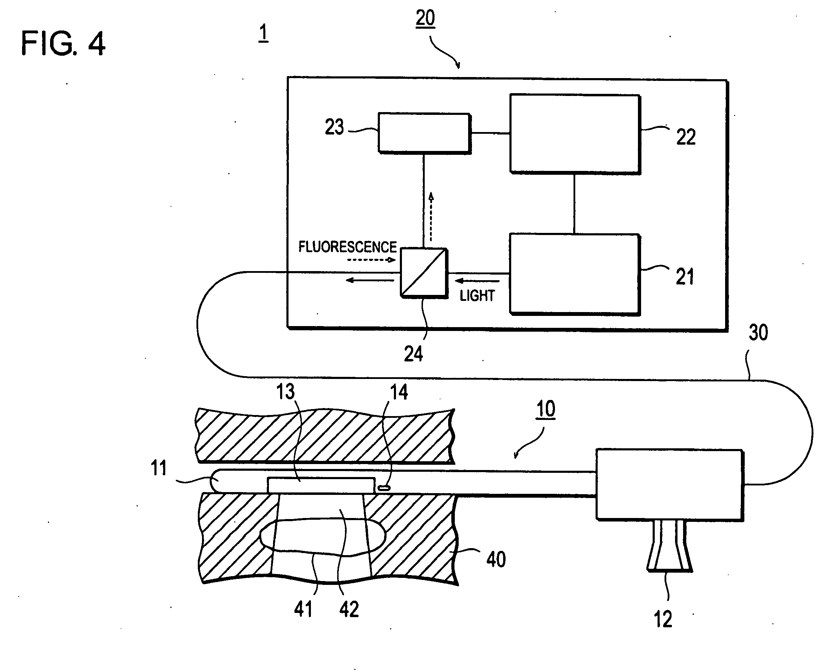

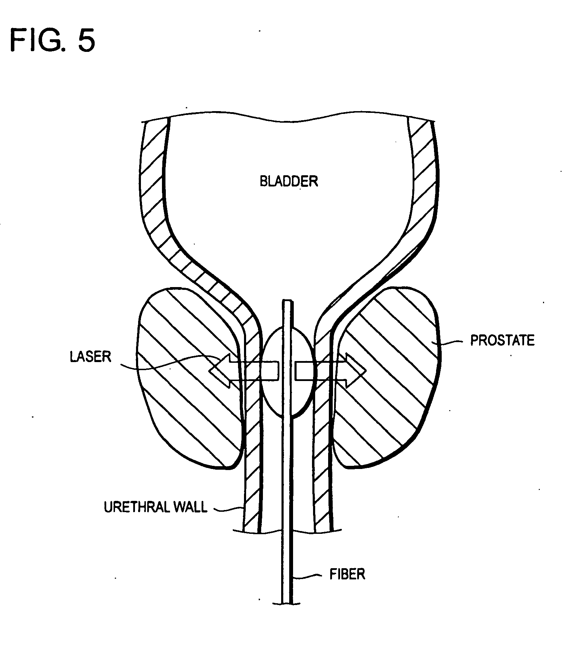

Fig. 4 is a block diagram showing schematic construction of photodynamic therapy equipment of the present invention. Fig. 5 is a schematic diagram showing photodynamic therapy equipment equipped with balloon on the tip of inserted part to the body.

As shown in Fig. 4 the photodynamic therapy equipment of the present invention comprises catheter 10, the tip of which is inserting into the body, the main unit of treatment apparatus 20 connected to the catheter 10, and the optical fiber (quartz fiber) 30 inserted into the catheter 10, connected to the irradiation part 13 in the catheter 10 in one end, and connected to the main unit of treatment apparatus 20 in another end.

The catheter 10 can be a conventionally used catheter and the diameter is not limited. The proper catheter depending on the lesioned part to be treated can be used. For example, the vascular catheter can be used for treatment of arteriosclerosis, and the urethral catheter can be used for treatment of prostate cancer or prostate hyperplasia.

The catheter 10 comprises the tip element 11 constructed to tube-like structure and supply opening 12, which is connected to the tip element 11, for supplying physiological saline containing photosensitive substance or oxygen when these are deficient with treatment. The tip element 11 herein indicates a part distance from several dozen centimeters.

In the tip element 11, the light irradiation element (irradiation means) 13 is placed. The light irradiation element 13 is connected with the light source 21 hereinbelow explained through the optical fiber 30 (quartz fiber). The light transmitted through the optical fiber 30 is irradiated from the window element placed on the side of tip element to the lesioned part 41.

In order to irradiate the light from the optical fiber 30 to the lateral direction, the light is refracted or scattered by using a prism or scattering substance. The tip of the optical fiber 30 may be processed to surface roughening. Further, light scattering substance such as alumina and silica may optionally be applied on the tip of the optical fiber 30, or as shown in Fig. 5, in case that the equipment of the present invention bears the balloon 15, these scattering substance may be contained in the balloon 15.

Since the irradiation should have little thermal effect on the penumbral tissues, the range of area within the lesioned part 41 irradiated by the light to the lateral direction is preferably 0.5 cm2 to 3 cm2. Even if the irradiation area is restricted locally, the lesion can be completely treated by rotating the catheter 10 depending upon the size of the lesioned part, changing the irradiation direction and performing multiple times of the irradiation to the lesioned part.

In case that the living body 40 to be treated is the blood vessel, for example, the lesioned part 41 is atheroma, and the preserved superficial part 42 is the fibrous membrane. Further in case that the living body 40 to be treated is the prostate tissue, the lesioned part 41 is benign or malignant tumor or inflammatory lesion, and the preserved superficial part 42 is the prostatic urinary tract.

In the tip element 11, the discharge opening 14 is placed close to the irradiation element 13. The discharge opening 14 spouts the photosensitive substance or oxygen supplied from the supply opening 12 into the living body 40.

The main unit of treatment apparatus 20 includes the light source 21, the control element 22 connected to the light source 21, the concentration determining device 23 connected to the control element 22, and the light separating element 24 connected to the light source 21 and the concentration determining device 23.

The light source 21 generates the high peak intensity pulsed light such as semiconductor laser beam, dye laser radiation and second harmonic waves of variable wavelength near-infrared laser beam.

The control element 22 adjusts the output peak intensity of the laser beam output from the light source 21 in order to make the output peak intensity of the laser beam to the predetermined range of the peak intensity proper to the treatment in the lesioned part 41.

The concentration determining device 23 is the device for detecting concentration of the photosensitive substance or oxygen in the body 40. The concentration determining device 23 detects the concentration of the photosensitive substance or oxygen contained in the lesioned part 41 by measuring changes of fluorescence or phosphorescence generated from the photosensitive substance in the treatment.

The optical fiber 30 transmits the light generated from the light source 21 to the catheter 10, and simultaneously transmits generated fluorescence or phosphorescence to the reverse direction to the main unit of treatment apparatus 20. The fluorescence or the phosphorescence transmitted from the catheter 10 is separated from the laser beam by the light separating element 24, selected the predetermined wavelength by the proper filter and transmitted to the concentration determining device 23.

The concentration determining device 23 is able to monitor amount of PDT drug and oxygen concentration by analyzing the fluorescence or the phosphorescence. For example, since when the porphyrin ring of the PDT drug is excited, the fluorescence is generated, amount of PDT drug can be measured by measuring the fluorescence. Further, since the phosphorescence is quenched depending on the oxygen concentration, the oxygen concentration can be measured by measuring the phosphorescence.

In addition, use of oxidative fluorescence indicator, in which fluorescence intensity is increased by an action of active oxygen, or phenomenon, in which the fluorescence reaction of the ruthenium complex is quenched depending on the oxygen concentration by immobilizing ruthenium complex to the optical fiber, may be utilized. Measurement of local oxygen partial pressure can be performed according to the description, J. M. Vanderkooi et al., J. Biol. Chem. 262 (12) : 5476-5482, Issue of April 25, 1987; Japan Chem. Soc. Ed. Exp. Chem. (Spectroscopy II), pp. 275-294, 1998; and Lichini M. et al. Chem. Commun., 19: pp. 1943-1944, 1999.

The concentration determining device 23 transmits the detection result to the control element 22. The control element 22 is able to change the light irradiation condition such as the light peak intensity generated by the light source 21 and the repetitive frequency based on detected photosensitive substance or oxygen concentration.

The control element 22 may optionally be connected with solenoid valve of tank (figure not shown). In this case, when deficiency of the photosensitive substance or oxygen concentration is detected by the concentration determining device 23, the magnetic valve is controlled to open, and the physiological saline which contains the photosensitive substance or oxygen concentration can also be supplied from the supply opening 12.

The optical fiber 30 used is, for example, having diameter about 0.05 to 0.6 mm. The optical fiber 30 may optionally be used any type, if it can be stored in the catheter 10 and transmitted the light energy from the light source 21.

In the above description, the same optical fiber 30 is used for transmitting the light from the light source 21 to the irradiation element 13 of the catheter 1, and transmitting the fluorescence or the phosphorescence from the body 40. However, the optical fiber transmitting the fluorescence or the phosphorescence can also be set independently and separately. In this case, the optical fiber for monitoring the fluorescence or the phosphorescence is connected directly to the concentration determining device 23.

In case of using the photodynamic therapy equipment 1 for treatment of prostate, the equipment may have the balloon 15 for the purpose of contacting the irradiation element 13 with the tissues. The balloon 15 is placed on near of the tip element 11 of the catheter 10.

When the equipment 1 is used for treatment of arteriosclerosis, the blood flow should be stopped in the lesion in the irradiation of the light beam. For that purpose, the balloon may be placed on the catheter 10.

The balloon placed in the equipment for arteriosclerosis can be the intravascular balloon used in the conventional catheter attached with balloon. The balloon 15 is expanded to stop the blood flow, and under such the condition, the light is irradiated to damage the lesion.

In this case, the balloon may optionally be equipped with the blood perfusion function, and the blood flow can be maintained by the blood flow perfusion function. Means for expanding balloon is not especially limited, and it can be achieved by supplying proper liquid or gas into the balloon. In this case, supply and drainage tube for liquid and gas may be equipped in the balloon.

Pressure of pressing the vascular wall by the expanding balloon is preferably at 0.2 to 1 kg/cm2. As explained hereinbefore, the irradiation element 13 may optionally be equipped to the balloon.

### (Use of the equipment of the present invention)

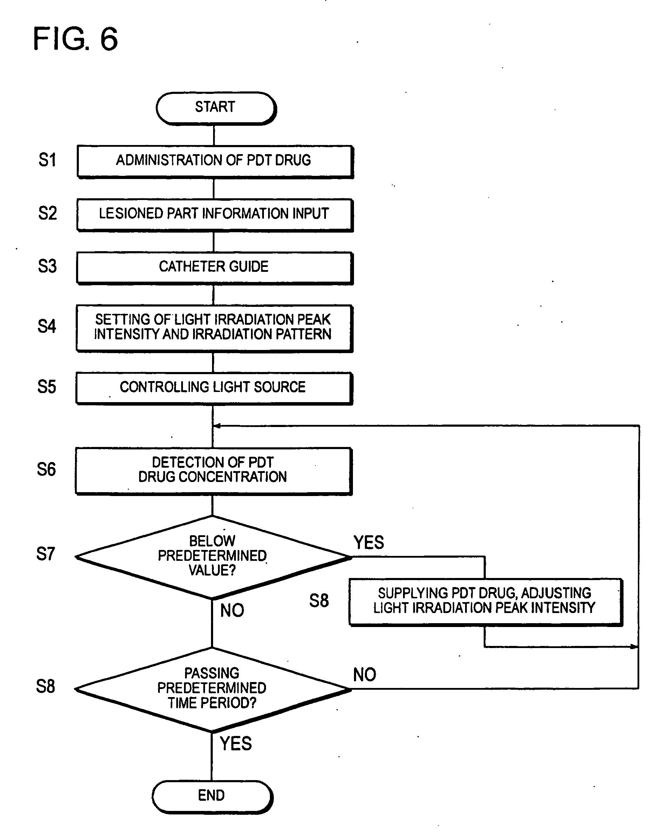

Method for use of the photodynamic therapy equipment 1 of the present invention hereinabove will be explained. Fig. 6 is a flow chart showing flow in use of equipment of the present invention.

At first, the photosensitive substance (PDT drug) is administered to the body previously to accumulate the photosensitive substance in the lesion (Step S1).

The preserved distance in the superficial part 41 is determined by the operator based on the information such as the depth and size of the lesion 41 obtained by previously performed ultrasound imaging, CT scan, plain roentgenography, MRI, etc., and the data is input to the control element 22 (Step S2). In this case, the shallower area than the depth of the lesion 41 is determined as the preservation distance.

The catheter 10 is inserted into the body and guided to the position close to the lesion 41 by the operator (Step S3). For example, in case of treating arteriosclerosis, the arterial catheter which can irradiate light is transferred close to the position where atheroma exists. Then the balloon is expanded to stop temporarily the flood flow.

The control element 22 set up the light irradiating peak intensity and the irradiating pattern based on the input preservation distance in Step S2 (Step S4) . The control element 22 stored previously data showing the correlationship between the preservation distance and the light irradiating peak intensity necessary for achieve the preservation, and the light irradiating peak intensity and the irradiation pattern are set based on such data.

The control element 22 irradiates the light from the light source 21 according to the setup light irradiating peak intensity and irradiation pattern (Step S5) to irradiate the light into the body from the tip of the catheter 10.

During irradiation of the light, concentration of the photosensitive substance contained in the lesion 41 is detected (Step S6). Measurement result is feedback to the control element 22, and data that the concentration of the photosensitive substance is below the predetermined value or not, is judged (Step S7).

In case of lowering the photosensitive substance below the predetermined value (Step S7: YES) , even if the irradiation of light is continued under the condition as it is, the therapeutic efficiency may be worse, then in order to obtain stable therapeutic efficiency, the control element 22 makes lower the light irradiation peak intensity or promotes supplying the physiological saline containing the photosensitive substance (Step S8). Then the process is returned to the treatment in Step S6.

In case not to lower the concentration of the photosensitive substance below the predetermined value (Step S7 : NO), data that the predetermined time course has passed or not is determined (Step S9).

In case that the treatment time course is not passed (Step S9: NO), the process is returned to the treatment of Step S7.

In case that the treatment time course has passed (Step S9: YES), the treatment procedure is terminated.

As explained, using the photodynamic therapy equipment 1, the lesion 41 alone can be treated by changing the irradiation peak intensity by the light source 3 together with considering the depth and size of the lesion 41. Namely, the light is allowed to pass with maintaining the high peak intensity for not to activate the photosensitive substance through the healthy superficial part 42, which is shallower than the lesion 41, and the light is irradiated to the lesion 41 at the level stronger than the level of attenuated light intensity in order to exhibit the peak intensity to exactly activate the photosensitive substance in the lesion 41, as a result the lesion 41 alone can be treated by preserving the superficial part. For example, in case that arteriosclerosis is treated, arteriosclerosis alone can be damaged without damaging the normal membrane covered on arteriosclerosis.

Since the concentration of the photosensitive substance is detected and the light peak intensity is reduced or the physiological saline containing the photosensitive substance is supplied depending on decrease of the concentration, the decrease of the therapeutic efficiency caused by deficiency of the photosensitive substance and the decrease in the efficiency of the treatment time period can be prevented.

In the method for use hereinabove, detection of the photosensitive substance concentration is explained, the method is not limited to that. The oxygen concentration in the lesion 41 of the body 40 can also be detected. Based on the result of detection of the oxygen concentration, the physiological saline containing oxygen is supplied and the light peak intensity can also be reduced optionally.

In the above method, the case that the catheter 10 is inserted into the body is explained, the method is not limited to that. The catheter 10 is attached on the skin without inserting the catheter into the body, the subcutaneous lesion can be treated.

The present invention can be applied to the treatment of prostate cancer or prostate hyperplasia. In that case, the urethral catheter which can irradiate the light, is inserted into the urinary tract, transfer the light irradiation element to the lesioned part, then the light is irradiated to the lesion from inside of the urinary tact. The high peak intensity pulsed light is irradiated by using the equipment of the present invention, as a result, only the part of prostate cancer or prostate hyperplasia can be damaged without damaging the normal urinary tract.

### (Method for controlling treatment depth by light irradiating condition)

The present invention can be used in a method for controlling treatment depth by changing the light irradiating condition such as peak intensity and repetition frequency in PDT. Changes in the light irradiating condition can be performed by the light generating device. In this case, equipment used in PDT can be the catheter like equipment hereinbefore explained or the equipment equipped with the light generating device which can irradiate the light from the outside of the body. Treatment of diseases developed in any places of the body can be performed according to the method for controlling the depth of the present invention by adjusting the treatment depth.

In case of deep treatment depth, the light with higher peak intensity is irradiated, and in case of shallow treatment depth, the peak intensity of irradiating light can be reduced. In case of deep treatment depth, pulsed light is preferable, and in case of shallow treatment depth, the pulsed light and continuous light can also be used. Especially, in case that the normal region which should not be damaged is found between the lesion, which is intended to be treated, and the light irradiating element, controlling the treatment depth becomes effective.

In case of large thickness of the normal region existing between the lesion and the light irradiating element, the light of the high peak intensity can optionally be irradiated, and in case of small thickness of the normal region, the light with slightly lowered peak intensity can be irradiated. Further, in case that the lesion to be treated is spread broadly from the superficial part to the deep part, the light with high peak intensity and the light with low peak intensity are irradiated in combination. The deep part can be treated by the light with high peak intensity and the superficial part can be treated by the light with low peak intensity. Further, since treatment efficiency can be differed not only by the light peak intensity but also by the repetition frequency of the light, the treatment efficiency can be improved by changing the repetition frequency.

The present invention is explained concretely by the following examples, but it is not limited by these examples.

### Example 1

### Control of treatment depth on light irradiation condition using arteriosclerotic athero model

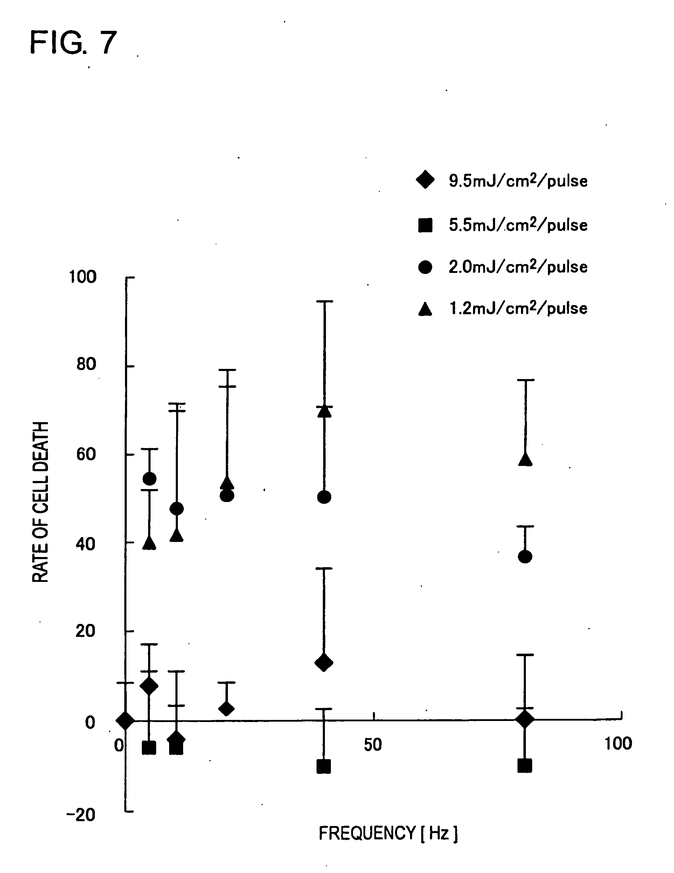

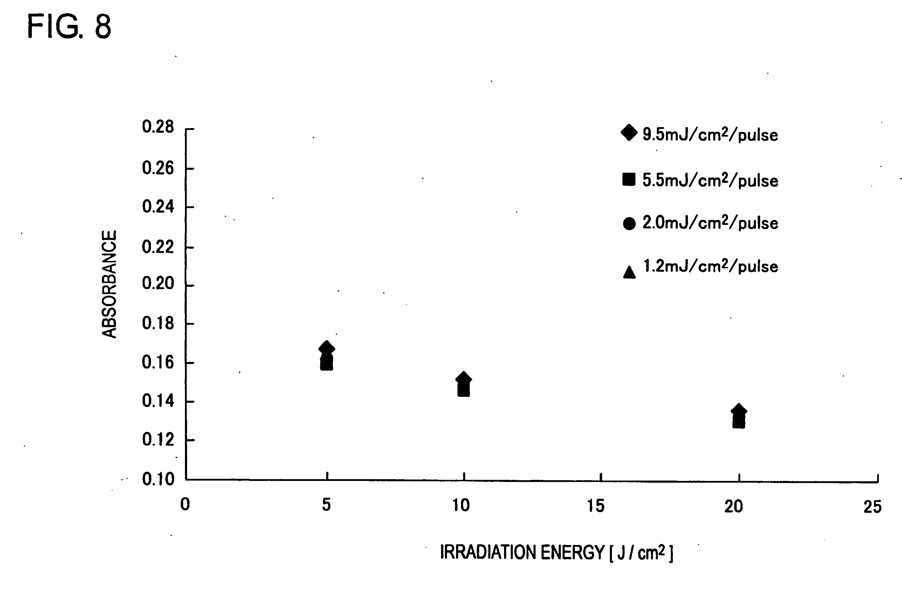

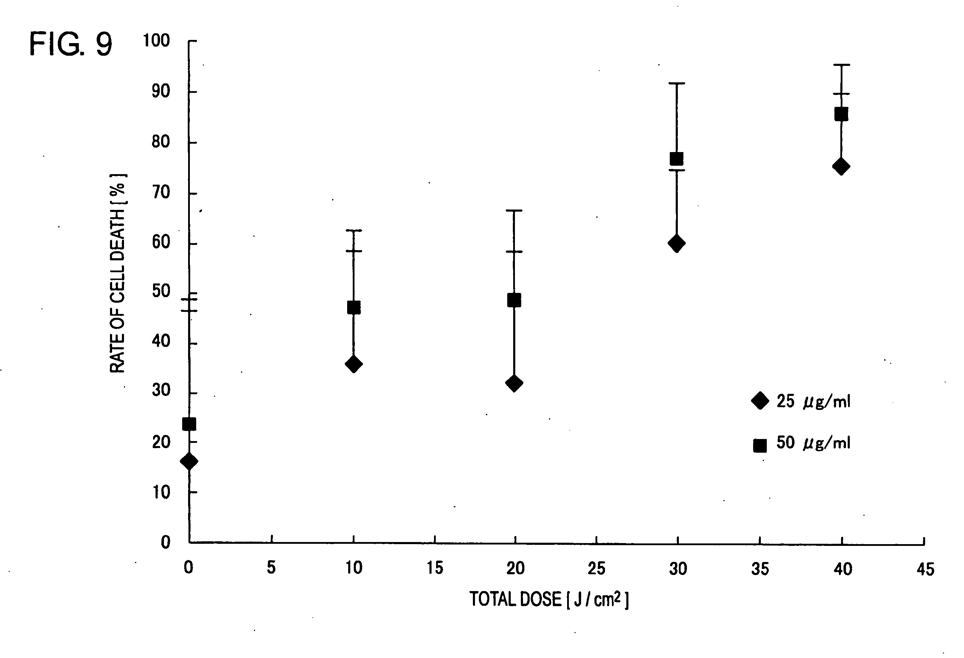

Fig. 7 is a graphical representation showing peak intensity and repetition frequency dependency of PDT effect. Fig. 8 is a graphical representation showing relationship between drug breaching and peak intensity. Fig. 9 is a graphical representation showing changes of rate of cell death when irradiation energy is changed.

In a treatment for attempting reduction of arteriosclerotic stenosed volume and maintaining perfusion blood flow rate, a method for controlling treatment depth by changing the light irradiation condition (peak intensity and frequency) for treating inside of athero alone and prevent damage of penumbral tissue was examined.

With regard to arteriosclerotic athero model, mouse derived macrophage like cell J774.1 was used. PDT was performed after contacting with the second generation photosensitive substance ATX-S10 (ATX-S10 Na (II) (K.K. Hikari Chemical Lab.)) with good excretion, concentration of 6 µg/ml for 24 hours.

Irradiation of the light was performed with the light source: excimer dye laser (EDL-1, Hamamatsu Photonix Co., wavelength 670 nm, pulse width 10 ns) ; pulse energy density 1.2 to 9.5mJ/cm² (corresponding to peak intensity 1.2 to 9.5×10⁸ W/cm²); and repetition frequency 5 to 80 Hz. PDT effect as evaluated by a rate of cell death using MTT after performing for 24 hours.

Result is shown in Fig. 7.

In a condition with high pulse energy density (high peak intensity), PDT effect almost disappeared. In a condition with low pulse energy density (low peak intensity), a rate of cell death at maximum 70% was obtained.

This might be caused by transient dissolved oxygen deficient. Presence of the optimum repetition frequency was also suggested.

In Fig. 8, changes of absorption of ATX-S10Na (II) at concentration 6 µg/ml with changing irradiation energy density are shown. Absorption depends on total irradiation energy (J/cm²) and not on peak intensity. Bleaching of drug was not a decreased rate of cell death at high peak intensity.

In Fig. 9, changes of a rate of cell death with changing the irradiation energy density are shown. Measurement was performed in concentration of administered photosensitive substance at 25 µg/ml and 50 µg/ml. As shown in Fig. 9, it can be understood that a rate of cell death is different, if the concentration of photosensitive substance is different regardless of the same irradiation energy density. Result indicates that rate of cell death can be adjusted by adjusting drug concentration.

Considering the organs, if the rate of cell death is too high, the organ may fall into organ failure, and the rate of cell death can be adjusted by adjusting concentration of drug in conformity to the type of organs.

This example indicates that the fibrous membrane can be preserved by irradiation of the high peak intensity. At the same time, it was indicated that treatment could be performed in the deep region (athero part) by means of decreased peak intensity caused by absorption. Further, it was indicated that rate of cell death could be adjusted by adjusting concentration of drug.

### Example 2

### Control of superficial part preservation range 1

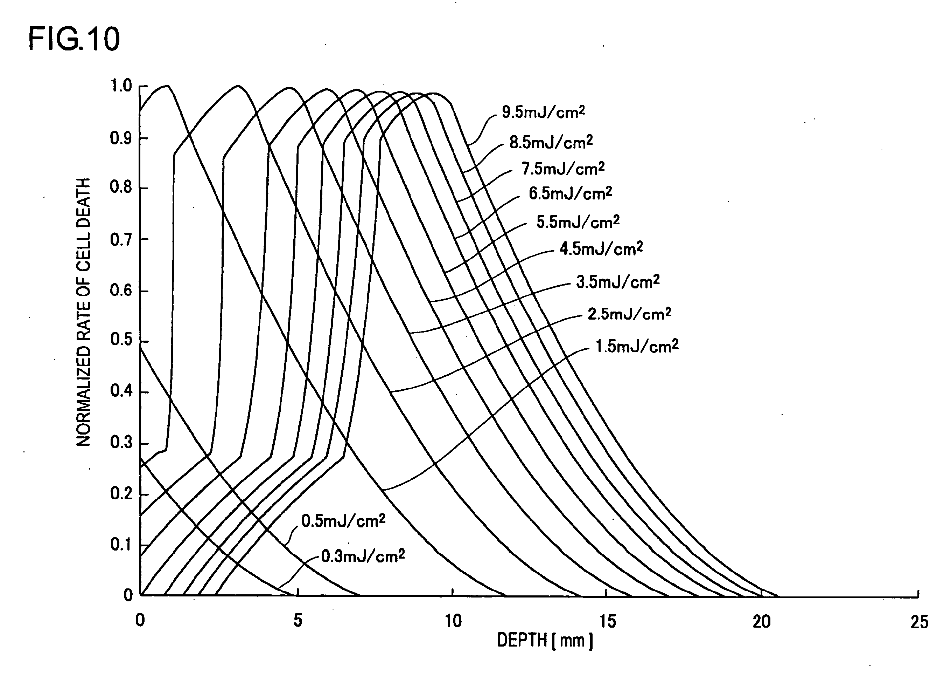

Fig. 10 is a graphical representation showing measurement result of rate of cell death to depth in each pulse energy density when pulse number is maintained constant.

In example 2, a rate of cell death on various depth with different pulse energy density under constant pulse number of irradiating light was measured.

10,000 pulses were irradiated under the condition of pulse energy density ranging from 0.3 mJ/cm² to 9.5 mJ/cm², with constant pulse width and repetition frequency of the irradiated pulsed light. The rate of cell death on the depth was measured. Result is shown in Fig. 10.

In the low pulse energy density at the range from 0.3 mJ/cm² to 1.5 mJ/cm², the rate of cell death was attenuated almost uniformly from the irradiated surface to the depth. Contrary to that, in the irradiation at pulse energy density 2.5 mJ/cm² or more, significantly low range of rate of cell death was remained in the superficial part.

A part of low rate of cell death is preserved due to condition under the cell fatality rate. Namely, the superficial preservation range, where the rate of cell death is lower than the cell fatality rate, and the fatal cell range, where the rate of cell death is higher than the cell fatality rate, positioned deeper than the superficial preservation range are formed. In the deeper area than the fatal cell range, again the rate of cell death is lower than the cell fatality rate, and the deep preservation range is formed.

Referring to Fig.10, it can be understood that as the peak intensity of the irradiating light is increased stepwise, the depth, where the rate of cell death becomes highest, goes to deeper, and the fatal cell range is formed in the deeper place. This indicates that the higher the pulse energy density of the irradiating light becomes, the broader the area, where the superficial preservation range is formed, occurs.

It is indicated that the depth for forming the fatal cell range can be controlled by controlling the pulse energy density of the irradiating light, in other word, by controlling the peak intensity of the irradiating light.

### Example 3

### Control of superficial part preservation range 2

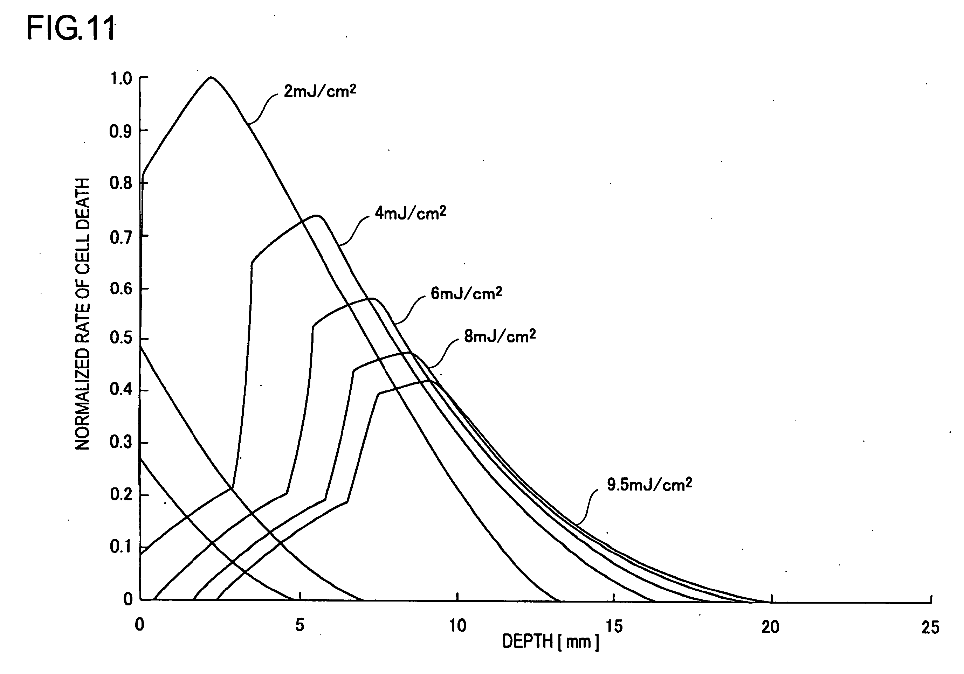

Fig. 11 is a graphical representation showing measurement result of rate of cell death to depth when amount of total irradiating energy is maintained constant.

In example 3, the rate of cell death to depth in each different pulse energy density with maintaining the total irradiation energy of irradiating light to be constant was measured.

Referring to the above example 2, an example of the superficial preservation treatment, when amount of the total irradiation energy is to be constant, is shown.

Irradiation was continued until the total amount of irradiation energy is reached to 40 J under the condition of pulse energy density ranging from 2 mJ/cm² to 9.5 mJ/cm², i.e. the range to form the superficial preservation range, with constant pulse width and repetition frequency of the irradiated pulsed light. The rate of cell death on the depth was measured. Result is shown in Fig. 11.

High rate of cell death can be achieved by irradiating with low pulse energy density rather than irradiating with high pulse energy density. Although the rate of cell death becomes lower in case that irradiation is performed with high pulse energy density rather than in case of performing irradiation with low pulse energy density, the fatal cell range is formed in the deeper place, namely the superficial preservation range is formed broadly.

As explained, the superficial preservation range can be controlled to make the range broader or narrower. This can be performed by controlling the irradiating pulse energy density with maintaining the irradiation energy to be constant, alternatively, by controlling the peak intensity of the irradiating light.

### Example 4

### Control of fatal cell range

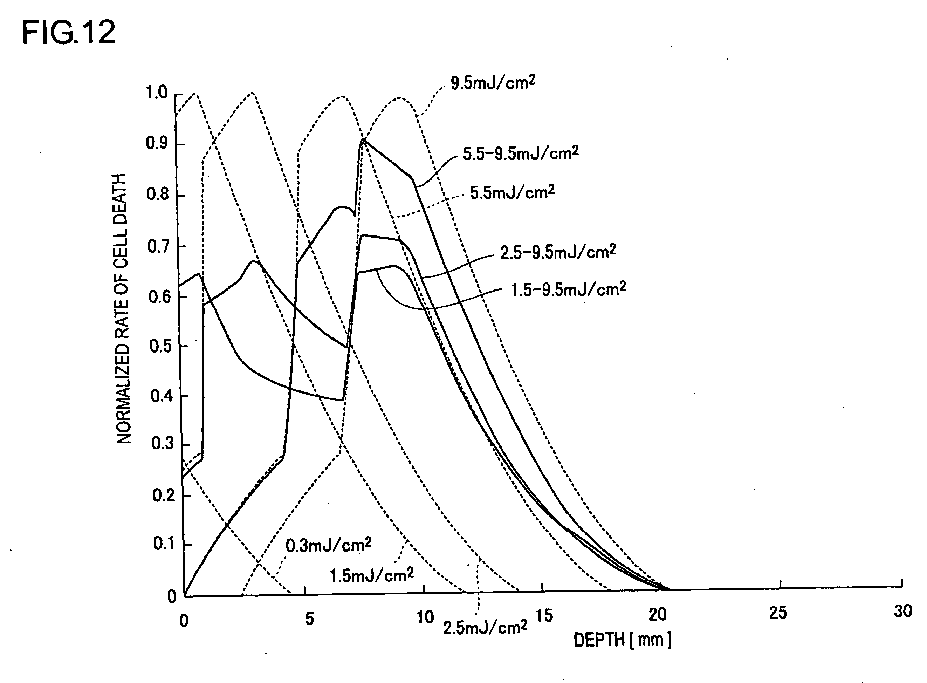

Fig. 12 is a graphical representation showing measurement result of rate of cell death to the depth when the pulse energy density is changed continuously or intermittently during irradiation.

In example 4, the rate of cell death to the depth was measured with maintaining the total number of irradiating pulse to be constant and changing the pulse energy density during the irradiation.

Example with controlling the fatal cell range was performed as follows. The peak intensity of light was maintained initially to the low peak intensity of 1.5 - 5.5 mJ/cm² and irradiated 5000 pulses, then changed to the high peak intensity of 9.5 mJ/cm² and irradiated 5000 pulses. Other conditions were the same as in examples hereinbefore. The rate of cell death to the depth was measured. Results are shown in Fig. 12. In Fig. 12, results of measurement in the case that the peak intensity was not changed during the experiment are also indicated.

Referring to Fig. 12, according to the results with changing the pulse energy density from 1. 5 mJ/cm² to 9. 5 mJ/cm² during the experiment, the superficial lesion was treated at first by the irradiation with low pulse energy density at 1.5 mJ/cm², subsequently the deep part was treated by the irradiation with high pulse energy density at 9.5 mJ/cm². Namely, the fatal cell range was formed to the broad range from the superficial to the deep part.

As a result of changing the pulse energy density from 2.5 mJ/cm² to 9.5 mJ/cm², and from 5.5 mJ/cm² to 9.5 mJ/cm², the peaks of rate of cell death were exhibited in two places of the superficial part and the deep part. As explained, a number of pulse irradiation is kept constant and the peak intensity is changed during the experiment, and distribution of the rate of cell death can be controlled. Namely, the fatal cell range can be formed to the broad range from the superficial to the deep part.

Further, the case that irradiation was performed with the pulse energy density of 9.5 mJ/cm² from the beginning to the end (21 in Fig.) and the case that irradiation was performed with the pulse energy density of 2.5 mJ/cm² at the beginning, subsequently irradiated with 9.5 mJ/cm² (23 in Fig.), are compared. The depth of the peak of the rate of cell death after changing to 9.5 mJ/cm² and the depth of the peak irradiated with the pulse energy at 9.5 mJ/cm² from the beginning to the end are almost same. Namely, it is indicated that the deepest part of the formed fatal cell range is almost same depth. In this regard, it can be understood that even if the peak intensity is changed to the different peak intensity from the halfway, the peak of the rate of cell death can be obtained with almost same depth when the irradiation is performed by each pulse energy density.

In the case irradiating with the pulse energy density at 9.5 mJ/cm² from the halfway, another one fatal cell range is formed in the shallower part during the course of irradiation at 2.5 mJ/cm². Consequently, it can also be understood that the fatal cell range is formed in the broader range rather than irradiating with only pulse energy density at 9. 5 mJ/cm².

As shown hereinabove, it is indicated that the fatal cell range can be formed in the broader range by combining with irradiation of different peak intensity rather than irradiating with the light of fixed peak intensity.

### Industrial applicability

As indicated in above examples, in case that the light irradiation condition was made to change in PDT, decrease in PDT efficiency was confirmed in the high peak intensity, and further in case that the frequency of the irradiating light was within constant range, the PDT efficiency was preferable. In case that PDT is performed to the biomedical tissues by changing the light irradiation condition using the equipment of the present invention, when the light with high peak intensity is irradiated, cells are not damaged in the superficial part where the peak intensity of light is high, and cells are damaged in the deep part, where the peak intensity of the light is decreased by absorption of energy with PDT drug, hemoglobin and water. As indicated, the therapeutic depth can be controlled by changing the irradiation condition of the irradiating light. Further, in case that the superficial part is the normal region and the deep part is the lesioned part, the superficial preservation therapy, wherein the deep lesioned part is damaged with remaining the normal superficial part, can be performed by using the equipment of the present invention.

## Claims

1. Photodynamic therapy equipment comprising:
an irradiation means (13) irradiating a pulsed light of the wavelength having the potential for activating the photosensitive substance, which is activated by the light having a peak intensity of a predetermined range but is almost not activated by the light having the peak intensity out of the predetermined range, a monitoring means (23) for monitoring at least one of the amounts of a PDT drug and oxygen concentration, and
a control means (22) controlling the condition of the irradiation of the light irradiated from the irradiation means (13),
**characterized in that**
a depth information of a lesioned part (41) is input into the control means (22) which is adapted to set up the light irradiating peak intensity based on the depth information so that the pulsed light is allowed to pass while maintaining the high peak intensity for not activating the photosensitive substance through the healthy superficial part (42), which is shallower than the lesioned part (41), and the pulsed light reaching to the lesion (41) at the level of attenuated light intensity in order to exhibit the peak intensity to exactly activate the photosensitive substance in the lesioned part (41), and,
the control means (22) is adapted to control the activation of the photosensitive substance by changing a irradiation condition of the light based on a result of the monitoring by the monitoring means (23), and control a rate of cell death caused by an action of the activated photosensitive substance in a direction of the depth in the body (40).

2. The photodynamic therapy equipment according to claims 1 wherein the irradiation condition of the light includes at least one of the peak intensity, wavelength, total irradiation time, total irradiation energy, pulse width and repetition frequency of the light.

3. The photodynamic therapy equipment according to claim 1 wherein the rate of cell death in the direction of the depth in the body (40) is high in a corresponding part of the body (40) and low in a superficial part (42) shallower than the corresponding part.

4. The photodynamic therapy equipment according to any of claims 1 to 3 wherein the rate of cell death in the direction of the depth in the body (40) is distributed high in a corresponding part of the body and low in the superficial part (42) located shallower than the corresponding part and in the deep part located deeper than the corresponding part.

5. The photodynamic therapy equipment according to claim 4 wherein the rate of cell death exceeds the cell fatality rate, which is impossible to regenerate cells, in the corresponding part of the body (40), and the rate of cell death is less than the cell fatality rate in the superficial part located shallower than the corresponding part and in the deep part located deeper than the corresponding part.

6. The photodynamic therapy equipment according to claim 5 wherein the control means (22) controls a range of the cell fatality rate in order that the rate of cell death is maintained to above the cell fatality rate by controlling the output power of the light.

7. The photodynamic therapy equipment according to claim 5 wherein the control means (22) controls the range of the cell fatality rate by keeping the total number of the irradiation pulses of the light irradiated from the light irradiation means constant, and controls the range of the cell fatality rate by controlling the peak intensity of the light.

8. The photodynamic therapy equipment according to claim 5 wherein the control means (22) controls the range of the cell fatality rate by keeping the total irradiation energy of the light irradiated from the light irradiation means constant, and controls the range of the cell fatality rate by controlling the peak intensity of the light.

9. The photodynamic therapy equipment according to claim 5 wherein the control means (22) controls the range of the cell fatality rate by changing continuously or intermittently the peak intensity of the light irradiated by the light irradiation means (13).

10. The photodynamic therapy equipment according to any of claims 1 to 9 comprising further a catheter (10) inserted into the position adjacent to the lesioned part (41) in the body (40), and guiding the light irradiation means (13) to the position adjacent to the lesioned part (41) by a guidance of the catheter (10).

11. The photodynamic therapy equipment according to claims 10 wherein the catheter (10) is a vascular balloon catheter.

12. The photodynamic therapy equipment according to claims 10 wherein the catheter (10) is an urethral catheter.

13. The photodynamic therapy equipment according to claim 1, wherein said control means (20) controls the depth in the body (40), where the photosensitive substance is activated, in the position adjacent to the lesioned part (41) by allowing the irradiation means (13) to irradiate the light having the high peak intensity in order that the light arriving at the deep-lying lesioned part (41) is to achieve the peak intensity of the predetermined range, and controls not to activate the photosensitive substance in the superficial part (42) of the body (40) positioned closer to the light irradiation means (13) than the lesioned part (41).

14. The photodynamic therapy equipment according to claim 13 wherein the control means (20) further controls the repetition frequency of the light irradiated by the irradiation means.

15. The photodynamic therapy equipment according to claim 13 or 14, wherein the light having the high peak intensity has the peak intensity of 10 kW/cm² or more which is below the threshold value generating the plasma in the surface of the body (40) by the light pulse irradiation, and the repetition frequency is 1 Hz to 1 kHz.

16. The photodynamic therapy equipment according to any of claims 13 to 15, wherein the control means (22) allows the irradiation means to irradiate the light having a low peak intensity lower than the high peak intensity by controlling the peak intensity of the light to the predetermined range at the superficial part (42), when the lesioned part located in the superficial part (42)is treated.

17. The photodynamic therapy equipment according to any of claims 13 to 16 comprising further a detection means (23) detecting at least one of the amounts of the photosensitive substance accumulated in the lesioned part (41) and the oxygen concentration of the lesioned part (41).

18. The photodynamic therapy equipment according to any of claims 13 to 17, wherein the light is selected from the group consisting of light generated from optical parametric oscillator, semiconductor laser beam, dye laser radiation and second harmonic waves of variable wavelength near-infrared laser beam.

19. The photodynamic therapy equipment according to any of claims 13 to 18 comprising further a catheter (10) inserting into the position adjacent to the lesioned part (41) in the body (40) and guiding the light irradiation means to the position adjacent to the lesioned part (41) by a guidance of the catheter (10).

20. The photodynamic therapy equipment according to claims 19 wherein the catheter (10) is a vascular balloon catheter.

21. The photodynamic therapy equipment according to claim 19 wherein the catheter (10) is an urethral catheter.

22. The photodynamic therapy equipment according to any of claims 13 to 21 wherein the control means (22) controls the depth in the body (40), where the photosensitive substance is activated, by maintaining constantly the total number of pulse of the light irradiated from the light irradiation means (13), and controlling the peak intensity of the light.

23. The photodynamic therapy equipment according to any of claims 13 to 21 wherein the control means (13) controls the depth in the body (40), where the photosensitive substance is activated, by keeping the total irradiation energy of the light irradiated from the light irradiation means (13) constant, and controlling the peak intensity of the light.

24. The photodynamic therapy equipment according to any of claims 13 to 21 wherein the control means (13) controls the area in the body (40), where the photosensitive substance is activated, by changing continuously or intermittently the peak intensity of the light irradiated from the light irradiation means (13).

## Patentansprüche

1. Photodynamisches Therapiegerät, das Folgendes umfasst:
ein Bestrahlungsmittel (13), das ein Impulslicht mit einer Wellenlänge ausstrahlt, die das Potential dafür hat, die lichtempfindliche Substanz zu aktivieren, die durch das Licht, das eine Spitzenintensität eines vorbestimmten Bereichs hat, aktiviert wird, aber durch das Licht, das die Spitzenintensität außerhalb des vorbestimmten Bereichs hat, nahezu nicht aktiviert wird, ein Überwachungsmittel (23) zum Überwachen wenigstens der Mengen eines PDT-Arzneimittels und/oder der Sauerstoffkonzentration, und
ein Steuerungsmittel (22), das den Zustand der Lichtstrahlung von dem Bestrahlungsmittel (13) steuert,
**dadurch gekennzeichnet, dass**
eine Tiefeninformation eines verletzten Teils (41) in das Steuerungsmittel (22) eingegeben wird, das dafür eingerichtet ist, die Spitzenintensität der Lichtbestrahlung auf der Grundlage der Tiefeninformation einzustellen, so dass ermöglicht wird, dass das Impulslicht hindurchgeht, während die hohe Spitzenintensität, um die lichtempfindliche Substanz nicht zu aktivieren, durch den gesunden oberflächlichen Teil (42), der flacher ist als der verletzte Teil (41), aufrechterhalten wird, und das Impulslicht die Verletzung (41) auf dem Niveau einer abgeschwächten Lichtintensität erreicht, so dass es die Spitzenintensität zeigt, um die lichtempfindliche Substanz in dem verletzten Teil (41) genau zu aktivieren, und
das Steuerungsmittel (22) dafür eingerichtet ist, die Aktivierung der lichtempfindlichen Substanz durch das Verändern eines Strahlungszustandes des Lichtes auf der Grundlage eines Ergebnisses der Überwachung durch das Überwachungsmittel (23) zu steuern und eine Rate des durch eine Wirkung der aktivierten lichtempfindlichen Substanz verursachten Zelltodes in einer Richtung der Tiefe in dem Körper (40) zu steuern.

2. Photodynamisches Therapiegerät nach Anspruch 1, wobei der Strahlungszustand des Lichtes wenigstens die Spitzenintensität, die Wellenlänge, die Gesamtstrahlungszeit, die Gesamtstrahlungsenergie, die Impulsbreite und/oder die Wiederholungsfrequenz des Lichtes einschließt.

3. Photodynamisches Therapiegerät nach Anspruch 1, wobei die Rate des Zelltodes in einer Richtung der Tiefe in dem Körper (40) in einem entsprechenden Teil des Körpers (40) hoch und in einem oberflächlichen Teil (42), der flacher ist als der entsprechende Teil, niedrig ist.

4. Photodynamisches Therapiegerät nach einem der Ansprüche 1 bis 3, wobei die Rate des Zelltodes in einer Richtung der Tiefe in dem Körper (40) in einem entsprechenden Teil des Körpers hoch verteilt und in dem oberflächlichen Teil (42), der flacher angeordnet ist als der entsprechende Teil, und in dem tiefen Teil, der tiefer angeordnet ist als der entsprechende Teil, niedrig ist.

5. Photodynamisches Therapiegerät nach Anspruch 4, wobei die Rate des Zelltodes in dem entsprechenden Teil des Körpers (40) die Zellsterblichkeitsrate, bei der es unmöglich ist, Zellen zu regenerieren, überschreitet und die Rate des Zelltodes in dem oberflächlichen Teil, der flacher angeordnet ist als der entsprechende Teil, und in dem tiefen Teil, der tiefer angeordnet ist als der entsprechende Teil, geringer ist als die Zellsterblichkeitsrate.

6. Photodynamisches Therapiegerät nach Anspruch 5, wobei das Steuerungsmittel (22) einen Bereich der Zellsterblichkeitsrate steuert, so dass die Rate des Zelltodes durch das Steuern der Ausgangsleistung des Lichtes oberhalb der Zellsterblichkeitsrate gehalten wird.

7. Photodynamisches Therapiegerät nach Anspruch 5, wobei das Steuerungsmittel (22) den Bereich der Zellsterblichkeitsrate durch das Konstanthalten der Gesamtzahl der Bestrahlungsimpulse des von dem Lichtbestrahlungsmittel ausgestrahlten Lichtes steuert und den Bereich der Zellsterblichkeitsrate durch das Steuern der Spitzenintensität des Lichtes steuert.

8. Photodynamisches Therapiegerät nach Anspruch 5, wobei das Steuerungsmittel (22) den Bereich der Zellsterblichkeitsrate durch das Konstanthalten der Gesamtstrahlungsenergie des von dem Lichtbestrahlungsmittel ausgestrahlten Lichtes steuert und den Bereich der Zellsterblichkeitsrate durch das Steuern der Spitzenintensität des Lichtes steuert.

9. Photodynamisches Therapiegerät nach Anspruch 5, wobei das Steuerungsmittel (22) den Bereich der Zellsterblichkeitsrate durch das fortlaufende oder unterbrochene Verändern der Spitzenintensität des durch das Bestrahlungsmittel (13) ausgestrahlten Lichtes steuert.

10. Photodynamisches Therapiegerät nach einem der Ansprüche 1 bis 9, das ferner einen Katheter (10), der in die Stellung angrenzend an den verletzten Teil (41) des Körpers (40) eingeführt wird, umfasst und das Lichtbestrahlungsmittel (13) durch eine Führung des Katheters (10) zu der Stellung angrenzend an den verletzten Teil (41) führt.

11. Photodynamisches Therapiegerät nach Anspruch 10, wobei der Katheter (10) ein Gefäß-Ballonkatheter ist.

12. Photodynamisches Therapiegerät nach Anspruch 10, wobei der Katheter (10) ein Harnröhrenkatheter ist.

13. Photodynamisches Therapiegerät nach Anspruch 1, wobei das Steuerungsmittel (20) die Tiefe in dem Körper (40), wo die lichtempfindliche Substanz aktiviert wird, steuert durch das Ermöglichen, dass das Bestrahlungsmittel (13) das Licht ausstrahlt, das die hohe Spitzenintensität hat, so dass das Licht, das den tiefliegenden verletzten Teil (41) erreicht, die Spitzenintensität des vorbestimmten Bereichs erreichen soll, und steuert, dass die lichtempfindliche Substanz in dem oberflächlichen Teil (42) des Körpers (40), der näher an dem Bestrahlungsmittel (13) angeordnet ist als der verletzte Teil (41), nicht aktiviert wird.

14. Photodynamisches Therapiegerät nach Anspruch 13, wobei das Steuerungsmittel (20) ferner die Wiederholungsfrequenz des durch das Bestrahlungsmittel ausgestrahlten Lichtes steuert.

15. Photodynamisches Therapiegerät nach Anspruch 13 oder 14, wobei das Licht, das die hohe Spitzenintensität hat, die Spitzenintensität von 10 kW/cm² oder mehr hat, die unter dem Schwellenwert liegt, der durch die Lichtimpulsbestrahlung das Plasma in der Oberfläche des Körpers (40) erzeugt, und die Wiederholungsfrequenz 1 Hz bis 1 kHz beträgt.

16. Photodynamisches Therapiegerät nach einem der Ansprüche 13 bis 15, wobei das Steuerungsmittel (22) ermöglicht, dass das Bestrahlungsmittel das Licht ausstrahlt, das eine niedrige Spitzenintensität hat, die niedriger ist als die hohe Spitzenintensität, durch das Steuern der Spitzenintensität des Lichtes auf den vorbestimmten Bereich an dem oberflächlichen Teil (42), wenn der in dem oberflächlichen Teil (42) angeordnete verletzte Teil behandelt wird.

17. Photodynamisches Therapiegerät nach einem der Ansprüche 13 bis 16, das ferner ein Erfassungsmittel (23) umfasst, das wenigstens die Mengen der lichtempfindlichen Substanz, die in dem verletzten Teil (41) angesammelt sind, und/oder die Sauerstoffkonzentration des verletzten Teils (41) erfasst.

18. Photodynamisches Therapiegerät nach einem der Ansprüche 13 bis 17, wobei das Licht ausgewählt ist aus der Gruppe, die aus Licht besteht, das von einem optischen parametrischen Oszillator, einem Halbleiter-Laserstrahl, Farbstoff-Laserstrahlung und zweiten harmonischen Wellen eines Nahe-Infrarot-Laserstrahls mit veränderlicher Wellenlänge erzeugt wird.

19. Photodynamisches Therapiegerät nach einem der Ansprüche 13 bis 18, das ferner einen Katheter (10), der in die Stellung angrenzend an den verletzten Teil (41) des Körpers (40) eingeführt wird, umfasst und das Lichtbestrahlungsmittel durch eine Führung des Katheters (10) zu der Stellung angrenzend an den verletzten Teil (41) führt.

20. Photodynamisches Therapiegerät nach Anspruch 19, wobei der Katheter (10) ein Gefäß-Ballonkatheter ist.

21. Photodynamisches Therapiegerät nach Anspruch 19, wobei der Katheter (10) ein Harnröhrenkatheter ist.

22. Photodynamisches Therapiegerät nach einem der Ansprüche 13 bis 21, wobei das Steuerungsmittel (22) die Tiefe in dem Körper (40), wo die lichtempfindliche Substanz aktiviert wird, durch das fortlaufende Aufrechterhalten der Gesamtzahl der Impulse des von dem Lichtbestrahlungsmittel (13) ausgestrahlten Lichtes und das Steuern der Spitzenintensität des Lichtes steuert.

23. Photodynamisches Therapiegerät nach einem der Ansprüche 13 bis 21, wobei das Steuerungsmittel (13) die Tiefe in dem Körper (40), wo die lichtempfindliche Substanz aktiviert wird, durch das Konstanthalten der Gesamtstrahlungsenergie des von dem Lichtbestrahlungsmittel (13) ausgestrahlten Lichtes und das Steuern der Spitzenintensität des Lichtes steuert.

24. Photodynamisches Therapiegerät nach einem der Ansprüche 13 bis 21, wobei das Steuerungsmittel (13) das Gebiet in dem Körper (40), wo die lichtempfindliche Substanz aktiviert wird, durch das fortlaufende oder unterbrochene Verändern der Spitzenintensität des durch das Bestrahlungsmittel (13) ausgestrahlten Lichtes steuert.

## Revendications

1. Equipement de thérapie photodynamique, comprenant:
un moyen d'émission (13) émettant de la lumière pulsée à la longueur d'onde ayant le potentiel d'activer la substance photosensible, qui est activée par de la lumière ayant une intensité maximale incluse dans une plage prédéterminée mais n'est presque pas activée par de la lumière ayant une intensité maximale hors de la plage prédéterminée,
un moyen de surveillance (23) pour surveiller au moins un paramètre parmi la quantité d'un médicament TPD et la concentration en oxygène, et
un moyen de commande (22) réglant les conditions d'émission de la lumière émise par le moyen d'émission (13),
**caractérisé en ce que**:
des informations de profondeur d'une partie lésée (41) sont introduites dans le moyen de commande (22) qui est conçu pour régler l'intensité maximale d'émission de lumière sur la base des informations de profondeur de telle manière que la lumière pulsée peut passer, tout en conservant une intensité maximale élevée pour ne pas activer la substance photosensible, à travers la partie superficielle saine (42) qui est moins profonde que la partie lésée (41), et la lumière pulsée atteint la lésion (41) à un niveau d'intensité lumineuse atténuée de façon à avoir une intensité maximale qui active exactement la substance photosensible dans la partie lésée (41), et
le moyen de commande (22) est conçu pour régler l'activation de la substance photosensible par modification des conditions d'émission de la lumière sur la base d'un résultat de la surveillance par le moyen de surveillance (23), et régler un taux de mort cellulaire causé par l'action de la substance photosensible activée dans une direction de profondeur d'un corps (40).

2. Equipement de thérapie photodynamique selon la revendication 1, dans lequel les conditions d'émission de la lumière comprennent au moins un paramètre parmi l'intensité maximale, la longueur d'onde, la durée d'émission totale, l'énergie lumineuse totale, la durée d'impulsion et la fréquence de répétition de la lumière.

3. Equipement de thérapie photodynamique selon la revendication 1, dans lequel le taux de mort cellulaire dans la direction de profondeur du corps (40) est élevé dans une partie correspondante du corps (40) et faible dans une partie superficielle (42) moins profonde que la partie correspondante.

4. Equipement de thérapie photodynamique selon l'une quelconque des revendications 1 à 3, dans lequel le taux de mort cellulaire dans la direction de profondeur du corps (40) est distribué élevé dans une partie correspondante du corps, et faible dans la partie superficielle (42) située à une plus petite profondeur que la partie correspondante et dans une partie profonde située à une plus grande profondeur que la partie correspondante.

5. Equipement de thérapie photodynamique selon la revendication 4, dans lequel le taux de mort cellulaire dépasse le taux de létalité cellulaire, auquel il est impossible que les cellules se régénèrent, dans la partie correspondante du corps (40), et le taux de mort cellulaire est inférieur au taux de létalité cellulaire dans la partie superficielle située à une plus petite profondeur que la partie correspondante et dans la partie profonde située à une plus grande profondeur que la partie correspondante.

6. Equipement de thérapie photodynamique selon la revendication 5, dans lequel le moyen de commande (22) règle une plage du taux de létalité cellulaire, dans laquelle le taux de mort cellulaire est maintenu au-dessus du taux de létalité cellulaire, par réglage de la puissance d'émission de la lumière.

7. Equipement de thérapie photodynamique selon la revendication 5, dans lequel le moyen de commande (22) règle la plage du taux de létalité cellulaire par maintien à une valeur constante du nombre total des impulsions d'émission de la lumière émise par le moyen d'émission de lumière, et règle la plage du taux de létalité cellulaire par réglage de l'intensité maximale de la lumière.

8. Equipement de thérapie photodynamique selon la revendication 5, dans lequel le moyen de commande (22) règle la plage du taux de létalité cellulaire par maintien à une valeur constante de l'énergie lumineuse totale de la lumière émise par le moyen d'émission de lumière, et règle la plage du taux de létalité cellulaire par réglage de l'intensité maximale de la lumière.

9. Equipement de thérapie photodynamique selon la revendication 5, dans lequel le moyen de commande (22) règle la plage du taux de létalité cellulaire par modification continue ou par intermittence de l'intensité maximale de la lumière émise par le moyen d'émission de lumière (13).

10. Equipement de thérapie photodynamique selon l'une quelconque des revendications 1 à 9, comprenant en outre un cathéter (10) introduit jusqu'à une position adjacente à la partie lésée (41) dans le corps (40), et guidant le moyen d'émission de lumière (13) jusqu'à la position adjacente à la partie lésée (41) par un guidage du cathéter (10).

11. Equipement de thérapie photodynamique selon la revendication 10, dans lequel le cathéter (10) est un cathéter à ballon vasculaire.

12. Equipement de thérapie photodynamique selon la revendication 10, dans lequel le cathéter (10) est un cathéter urétral.

13. Equipement de thérapie photodynamique selon la revendication 1, dans lequel ledit moyen de commande (20) règle la profondeur dans le corps (40), à laquelle la substance photosensible est activée, à la position adjacente à la partie lésée (41) en permettant au moyen d'émission (13) d'émettre de la lumière ayant une intensité maximale élevée de telle manière que la lumière arrivant au niveau de la partie lésée (41) située en profondeur atteigne l'intensité maximale incluse dans la plage prédéterminée, et n'active pas la substance photosensible dans la partie superficielle (42) du corps (40) située plus près du moyen d'émission de lumière (13) que la partie lésée (41).

14. Equipement de thérapie photodynamique selon la revendication 13, dans lequel le moyen de commande (20) règle en outre la fréquence de répétition de la lumière émise par le moyen d'émission.

15. Equipement de thérapie photodynamique selon la revendication 13 ou 14, dans lequel la lumière ayant une intensité maximale élevée a une intensité maximale de 10 kW/cm² ou plus qui est inférieure à la valeur seuil générant un plasma à la surface du corps (40) par l'émission de lumière pulsée, et la fréquence de répétition est de 1 Hz à 1 kHz.

16. Equipement de thérapie photodynamique selon l'une quelconque des revendications 13 à 15, dans lequel le moyen de commande (22) permet au moyen d'émission d'émettre la lumière ayant une intensité maximale faible inférieure à l'intensité maximale élevée par réglage de l'intensité maximale de la lumière dans la plage prédéterminée au niveau de la partie superficielle (42), quand la partie lésée traitée se trouve dans la partie superficielle (42).

17. Equipement de thérapie photodynamique selon l'une quelconque des revendications 13 à 16, comprenant en outre un moyen de détection (23) détectant au moins un paramètre parmi la quantité de la substance photosensible accumulée dans la partie lésée (41) et la concentration en oxygène de la partie lésée (41).

18. Equipement de thérapie photodynamique selon l'une quelconque des revendications 13 à 17, dans lequel la lumière est choisie dans le groupe comprenant la lumière générée par un oscillateur paramétrique optique, le faisceau d'un laser à semi-conducteur, le rayonnement d'un laser à colorant et l'onde de deuxième harmonique d'un faisceau laser proche infrarouge à longueur d'onde variable.

19. Equipement de thérapie photodynamique selon l'une quelconque des revendications 13 à 18, comprenant en outre un cathéter (10) introduit jusqu'à la position adjacente à la partie lésée (41) dans le corps (40), et guidant le moyen d'émission de lumière jusqu'à la position adjacente à la partie lésée (41) par un guidage du cathéter (10).

20. Equipement de thérapie photodynamique selon la revendication 19, dans lequel le cathéter (10) est un cathéter à ballon vasculaire.

21. Equipement de thérapie photodynamique selon la revendication 19, dans lequel le cathéter (10) est un cathéter urétral.

22. Equipement de thérapie photodynamique selon l'une quelconque des revendications 13 à 21, dans lequel le moyen de commande (22) règle la profondeur dans le corps (40), à laquelle la substance photosensible est activée, par maintien à une valeur constante du nombre total d'impulsions de la lumière émise par le moyen d'émission de lumière (13) et réglage de l'intensité maximale de la lumière.

23. Equipement de thérapie photodynamique selon l'une quelconque des revendications 13 à 21, dans lequel le moyen de commande (13) règle la profondeur dans le corps (40), à laquelle la substance photosensible est activée, par maintien à une valeur constante de l'énergie lumineuse totale de la lumière émise par le moyen d'émission de lumière (13) et réglage de l'intensité maximale de la lumière.

24. Equipement de thérapie photodynamique selon l'une quelconque des revendications 13 à 21, dans lequel le moyen de commande (13) règle la zone dans le corps (40), dans laquelle la substance photosensible est activée, par modification continue ou par intermittence de l'intensité maximale de la lumière émise par le moyen d'émission de lumière (13).
